(19) Europäisches Patentamt
European Patent Office
Office européen des brevets

(11) Veröffentlichungsnummer: **0 103 823**
**B1**

(12) # EUROPÄISCHE PATENTSCHRIFT

(45) Veröffentlichungstag der Patentschrift: **20.04.88**

(51) Int. Cl.⁴: **C 12 Q 1/48**

(21) Anmeldenummer: **83108912.3**

(22) Anmeldetag: **09.09.83**

(54) **Reagenz und Verfahren zur Bestimmung von gamma-Glutamyltransferase.**

(30) Priorität: **17.09.82 DE 3234478**

(43) Veröffentlichungstag der Anmeldung: **28.03.84 Patentblatt 84/13**

(45) Bekanntmachung des Hinweises auf die Patenterteilung: **20.04.88 Patentblatt 88/16**

(84) Benannte Vertragsstaaten: **AT BE CH DE FR GB IT LI LU NL SE**

(56) Entgegenhaltungen:
**FR - A - 2 509 324**
**GB - A - 2 034 690**
**US - A - 4 167 449**

(73) Patentinhaber: **Boehringer Mannheim GmbH, Sandhofer Strasse 116, D-6800 Mannheim 31 (DE)**

(72) Erfinder: **Deneke, Ulfert, Dr.rer.nat, Birkenweg 5, D-6149 Rimbach-Zotzenbach (DE)**
Erfinder: **Nagel, Rolf, Am Bildstock 51, D-6842 Bürstadt (DE)**
Erfinder: **Rittersdorf, Walter, Dr.rer.nat., Kasselerstrasse 6, D-6800 Mannheim 31 (DE)**

**EP 0 103 823 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

## Beschreibung

Die Erfindung betrifft ein Reagenz und ein Verfahren zur Aktivitätsbestimmung des Enzyms γ-Glutamyltransferase (γ-GT, γ-Glutamyl-transpeptidase, EC Nr. 2.3.2.2) in Vollblut, Plasma, Serum, biologischen Flüssigkeiten oder anderem Material, bei dem geeignete γ-Glutamylarylamide als Substrat eingesetzt werden und das bei der γ-GT-Reaktion mit einem Akzeptormolekül freigesetzte Arylamin mittels einer geeigneten Kupplungskomponente und einem geeigneten Oxidationsmittel in einem Farbstoff überführt wird, dessen Bildung kinetisch verfolgt werden kann.

Seit der Entdeckung der γ-GT im Humanserum hat dieses Enzym insbesondere in der Leberdiagnostik eine grosse Bedeutung erlangt (H.U. Bergmeyer, Methoden der enzymatischen Analyse, Band I, Verlag Chemie, Weinheim 1974, Seite 14–30 und Seite 757). Zur Bestimmung der Aktivität der γ-GT in einer Probe bestimmt man üblicherweise die Geschwindigkeit der Reaktion eines γ-Glutamylamids mit Glycylglycin als Akzeptor unter Bildung von γ-Glutamylglycylglycid und Amin. Diese Bestimmungsmethoden lassen sich in zwei Klassen einteilen, eimmal in die bei denen das freigesetzte Amin selbst ein chromogenes Substrat ist und zum anderen in solche, bei denen das freigesetzte Amin erst durch Folgereaktionen in eine farbige Verbindung überführt wird.

Zur ersten Gruppe gehören verschiedene Nitroanilinderivate, die am Aminrest mit einem γ-Glutamylrest verknüpft sind. Durch die Übertragung des γ-Glutamylrestes auf das Akzeptormolekül wird das Nitroanilin freigesetzt, dessen Extinktionsmaximum gegenüber der Ausgangsverbindung bathochrom verschoben ist, so dass die Farbzunahme bei geeigneter Wellenlänge im Gelb- oder Rotbereich gemessen werden kann. Als chromogene Substrate für γ-GT sind vor allem γ-Glutamyl-p-nitroanilid und γ-Glutamyl-p-nitroanilid-3-carbonsäure bekannt geworden. Weniger gebräuchlich ist γ-Glutamyl-p-nitroanilid-3-sulfonsäure. Diese Substrate erlauben kinetische Messungen und Einpunktmessungen.

Zur zweiten Gruppe gehören die Substrate γ-Glutamyl-anilin, γ-Glutamyl-α-naphthylamin, γ-Glutamyl-β-naphthylamin γ-Glutamyl-p-hydroxyanilin oder deren unterschiedlich substituierten Derivate. Diese werden so eingesetzt, dass das in der γ-GT-Reaktion freigesetzte Arylamin mittels oxidativer Kupplung, Kondensation mit aromatischen Aldehyden oder durch Diazoniumsalzkupplung in einen messbaren Farbstoff überführt wird. Dabei wird die γ-GT-Reaktion für eine bestimmte Zeit durchgeführt und dann unter Zugabe von Säure oder Lauge die Farbreaktion durchgeführt, wobei die γ-GT-Reaktion gestoppt wird (Einpunktmessung). Siehe z.B. DAS 2 920 292, DAS 2 338 043). Eine kinetische Messung ist nach dieser Methode nicht möglich, da die Freisetzung des Amins und die Farbbildung nicht unter gleichen Bedingungen durchgeführt werden können.

Weitere in der Literatur beschriebene Bestimmungsmethoden der γ-GT haben keine praktische Bedeutung erlangt, weil sie aufwendig und schwierig zu handhaben sind.

Den oben aufgeführten Verfahren haften nun schwerwiegende Mängel an, die eine Anwendung besonders dann einschränken, wenn die γ-GT-Reaktion auf Teststreifen durchgeführt werden soll. Mit dem Auge ist eine Auswertung nämlich nur dann möglich, wenn die Lichtabsorption im genügend langwelligen Bereich stattfindet. Auch wenn Teststreifen remissionsphotometrisch ausgewertet werden sollen, muss Licht mit einer Wellenlänge von über 565 nm eingesetzt werden, da biologisches Material wie Plasma, Serum oder Urin eine Reihe Substanzen enthalten, die im kürzerwelligen Bereich absorbieren und damit die Messung negativ beeinflussen würden. Die bisher bekanntgewordenen chromogenen Substrate der γ-GT können jedoch höchstens bis 500 nm gemessen werden. Hier absorbiert besonders Bilirubin noch sehr stark, so dass selbst im photometrischen Test, der ja eine Verdünnung der Probe beinhaltet, oft der gesamte Messbereich des Photometers durch die Eigenabsorption der Probe verbraucht wird. Weiter überlappen die Spektren der γ-glutamylierten Substrate und des freigesetzten Chromogens meistens so stark, dass nur in der Bandenflanke des Chromogens gemessen werden kann, oder die Anfangsextinktion des Reagenz bereits sehr hoch ist. Das bedeutet, dass besonders hohe Anforderungen an die Qualität des Photometers zu richten sind und die Empfindlichkeit der Methoden eingeschränkt ist. Vorteilhaft an den chromogenen Substraten ist jedoch, dass sie eine kinetische Messung zulassen. Das ist besonders für Teststreifen von Bedeutung, weil hier praktisch nur kinetische Messungen einfach und sinnvoll remissionsphotometrisch ausgewertet werden können.

Die durch die oxidative Kupplung, durch Aldehydkondensation oder Diazoniumsalzkupplung erhaltenen Farbstoffe in den bekannten γ-GT-Testen sind in der Regel rot, violett oder blau und haben daher keine Probleme mit dem Probenleerwert. Hier handelt es sich jedoch ausschliesslich um Endpunktbestimmungen und diese lassen sich wiederum in Teststreifen nicht vernünftig realisieren.

Aufgabe der vorliegenden Erfindung war es demnach, einen γ-GT-Test zu schaffen, bei dem eine Farbstoffbildung kinetisch gemessen werden kann, wobei der gebildete Farbstoff sein Absorptionsmaximum bei über 565 nm, hat. Die Aufgabe wird erfindungsgemäss dadurch gelöst, dass ein aus einem γ-Glutamyl-arylamid freigesetztes Arylamin gleichzeitig durch oxidative Kupplung mit geeigneten Oxidationsmitteln und einer geeigneten Kupplungskomponente zu einem Farbstoff mit einem Absorptionsmaximum von über 565 nm gekuppelt wird, dessen Bildung kinetisch verfolgt werden kann und die Reaktion auch auf Teststreifen angewendet werden kann.

Bereits in den ersten Arbeiten zu der Charakterisierung der γ-GT ist beschrieben, dass es sich um ein Glycoprotein handelt, das gleichzeitig essentielle SH-Gruppen enthält (A. Szewczuk und T.

Baranowski, Biochem. Z. 338, 317–329 (1963)). Solche Verbindungen werden durch Oxidationsmittel normalerweise irreversibel verändert. Für das mit Perjodat behandelte Enzym ist diese Inhibierung bzw. Denaturierung in der zitierten Arbeit bereits beschrieben. Dies ist vermutlich einer der Gründe, warum alle bisher beschriebenen auf der oxidativen Kupplung beruhenden γ-GT-Reaktionen lediglich als Abstoppversion durchgeführt werden. Ein weiterer Grund ist sicherlich, dass im pH-Optimum der γ-GT, das heisst bei pH 6–10, insbesondere pH 7,5–8,5, die üblicherweise verwendeten Oxidationsmittel für die oxidative Kupplung nicht oder nur langsam reagieren. Da die Kupplungsreaktion aber schnell gegenüber der Spaltungsreaktion sein muss, um nicht für die kinetische Bestimmung geschwindigkeitsbestimmend zu sein, wurde bisher die oxidative Kupplung entweder im sauren oder alkalischen Medium durchgeführt, nachdem die Spaltung abgelaufen war.

Es muss daher höchst überraschen, dass es Oxidationsmittel gibt, die bei pH 6–10 die Amine so schnell oxidieren, dass man die Aktivität der γ-GT durch eine Kupplungsreaktion kinetisch messen kann und trotzdem das γ-GT-Enzym weder am Zuckeranteil noch an den SH-Gruppen irreversibel schädigen. Aus einer grossen Zahl von Oxidationsmitteln sind Ferricyanid und $H_2O_2$/POD bzw. Peroxydbildner/POD als bisher allein brauchbar gefunden worden. Ersteres wird bevorzugt.

Als Substrat können die γ-Glutamylverbindungen von Anilin, Naphthylamin, 2-Hydroxy-4-aminobenzoesäure und andere Verbindungen eingesetzt werden, die bei der γ-GT-Reaktion ein kupplungsfähiges Substrat liefern. Insbesondere geeignet sind Verbindungen der folgenden Formel I

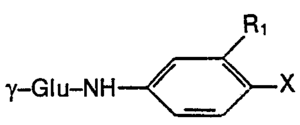
(I)

in der

X die Gruppe $NR_2R_3$ oder $-O-R_2$

$R_1$ Wasserstoff, eine Alkoxy-, Alkyl- oder Arylgruppe mit bis zu 8 C-Atomen, Chlor, Brom, Jod oder die Gruppe

$NR_2R_3$, $-SO_3R_2$, $-COOR_2$ und

$R_2$ und $R_3$ gleich oder verschieden sind und Wasserstoff, eine Alkyl- oder Arylgruppe mit bis zu 8 C-Atomen, die auch durch OH, Alkoxy, $-CO_2H$, $SO_3H$ substituiert sein kann darstellen.

Entsprechende Verbindungen und Verfahren zu ihrer Herstellung sind beispielsweise in der DE-OS 2 823 342 beschrieben.

Besonders bevorzugt wird das bisher in der Literatur nicht beschriebene Substrat γ-Glutamyl-p-phenylendiamin-3-carbonsäure, welches am besten durch Reduktion von γ-Glutamyl-4-nitroanilin-3-carbonsäure zugänglich ist.

Als Kupplungskomponenten eignen sich die für diese Reaktion bekannten Substrate, das heisst, aromatische Amine oder Phenole wie sie u.a. in der vorbezeichneten DE-OS 2 823 342 aufgeführt sind.

Besonders geeignet sind Verbindungen der Formel II

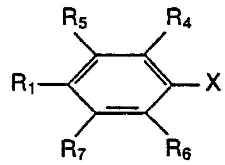
(II)

in der

X eine $NR_2R_3$ oder $-O-R_3$-Gruppe,

$R_1$ Wasserstoff oder Chlor, wenn $R_4$ und/oder $R_6$ auch Chlor sind,

$R_2$ und $R_3$ gleich oder verschieden sind und

Wasserstoff, eine Alkyl-, Aralkyl- oder Arylgruppe mit bis zu 8 C-Atomen, die gegebenenfalls auch durch Hydroxy, Alkoxy mit bis zu 5 C-Atomen, $-CO_2H$, $SO_3H$ substituiert sein kann,

$R_4$ und $R_6$ Wasserstoff, niedrig Alkyl mit bis zu 5 C-Atomen Chlor, Brom, Jod, $-COOR_2$, $SO_3R_2$, $OR_2$, $NR_2R_3$ wobei $R_2$ und $R_3$ die obige Bedeutung haben

$R_5$ und $R_7$ Wasserstoff oder eine Alkylgruppe mit bis zu 5 C-Atomen bedeuten, wobei

$R_4$ und $R_5$ beziehungsweise $R_6$ und $R_7$ zusammen mit dem Benzolring auch ein Naphthyl- oder Anthrylgerüst bilden können.

Die Alkylgruppen in den vorstehenden Verbindungen haben bis zu 6, vorzugsweise 1–3 C-Atome. Besonders bevorzugt ist die Methylgruppe.

Besonders bevorzugt sind z.B. 2,3-Xylenol, Diethylmetanilsäure, N-Ethyl-N-(3′-sulfobenzol)-anilin und N-Methylanthranilsäure.

Die erfindungsgemässen Reagenzien können neben den für die eigentliche Reaktion notwendigen Bestandteilen γ-Glutamylarylamid, Akzeptor (Glycylglycin), Kupplungskomponente, Oxidationsmittel und Puffersubstanz pH 6–10, noch Aktivatoren (z.B. Magnesiumsalze), Netzmittel, Stabilisierungsmittel, Verdickungsmittel und andere übliche Hilfsstoffe enthalten. Der Test kann entweder wie üblich in einer Küvette oder auch auf einem saugfähigen Träger (Teststreifen) durchgeführt werden, der mit den Reagenzien imprägniert ist.

Für einen üblichen Testansatz in einer Standard-Küvette mit 10 mm Schichtdicke und einem Testvolumen von 2–3 $cm^3$ wird eine Blut- oder Serummenge von 20–100 µl benötigt, bei Mikro-Küvetten entsprechend weniger. Die Reagenzlösung hat etwa die folgende Zusammensetzung jeweils in mmol/l Lösung:

| | |
|---|---|
| 1 –100 | γ-Glutamylarylamid |
| 10 –200 | Akzeptormolekül (z.B. Glycylglycin) |
| 1 –200 | Kupplungskomponente |
| 0,1–100 | Oxidationsmittel |
| 10 –200 | Puffer pH 6–10 |

ferner können noch

| | |
|---|---|
| 0– 10 | Aktivator (z.B. ein Magnesiumsalz) |
| 0– 10 | Netzmittel |
| 0– 50 | Stabilisierungsmittel |
| 0–100 | Verdickungsmittel |

enthalten sein.

Für den Test auf saugfähigem Träger kann ein Tropfen Serum oder Plasma (10–50 µl) auf den trockenen, mit Reagenzien imprägnierten Träger aufgebracht werden.

Wegen der geringen Schichtdicke, die zur Auswertung zur Verfügung steht, sollten die zur Imprägnierung verwendeten Reagenzlösungen etwas konzentrierter sein als beim Küvettentest. Folgende Zusammensetzungen in mmol/l sind geeignet:

| | |
|---|---|
| 5– 100 | γ-Glutamylarylamid |
| 50– 500 | Glycylglycin |
| 10– 500 | Kupplungskomponente |
| 1– 500 | Oxidationsmittel |
| 50–1000 | Puffer pH 6–10, insbesondere 7,5–8,5 |

sowie die für den Küvettentest angegebenen sonstigen Stoffe in der angegebenen Menge. Die brauchbaren γ-Glutamylarylamide, Kupplungskomponenten und Oxidationsmittel sind vorstehend bereits aufgeführt. Als Puffer können alle für den angegebenen pH-Bereich üblichen schwach alkalischen Puffer verwendet werden. EDTA, Trispuffer, 2-Amino-2-methylpropan-1,3-diol und ähnliche werden bevorzugt, da sie gleichzeitig auch noch Schwermetall-komplexierende Eigenschaften haben. Als Aktivatoren für das Enzym werden vorteilhaft Magnesiumsalze wie $MgCl_2$ etc. in geringer Menge zugesetzt. Die folgenden Beispiele sollen die Erfindung näher erläutern, ohne sie einzuschränken.

Beispiel 1

Küvettentest für γ-GT

| | |
|---|---|
| Tris. HCL, pH 8,0 | 100 mmol/l |
| γ-Glutamyl-p-phenylendiamin-3-carbonsäure | 10 mmol/l |
| Glycylglycin | 75 mmol/l |
| N-Methylanthranilsäure | 50 mmol/l |
| $K_3[Fe(CN)_6]$ | 0,5 mmol/l |
| Lösungsmittel: Wasser | |

Zu 3 ml dieser Lösung werden 50 µl Humanserum gegeben und bei einer Schichtdicke von 10 mm und einer Wellenlänge von 600–800 nm gemessen.

V = 3,05 ml, v = 0,05,T 37 °C, λ = 630 nm, ε = 18,4 $cm^2$/mmol

$$\frac{U}{1} = \frac{V \cdot \Delta E/\text{min. } 1000}{\varepsilon \cdot v}$$

| U/1 | ΔE/min. |
|---|---|
| 0 | 0 |
| 10 | 3 |
| 20 | 6 |
| 50 | 15 |
| 100 | 30 |
| 200 | 60 |
| 500 | 150 |

Beispiel 2

Teststreifen für γ-GT

Zur Herstellung von Teststreifen werden 2 Papiere imprägniert

A) Substratpapier

| | |
|---|---|
| Tris, pH 7,6 | 200 mmol/l |
| N-Methylanthranilsäure | 100 mmol/l |
| Glycylglycin | 250 mmol/l |
| EDTA-$Na_2$ | 100 mmol/l |
| γ-Glutamyl-p-phenylendiamin-3-carbonsäure | 20 mmol/l |
| in Wasser | |

Mit dieser Lösung wird ein Papier geeigneter Dicke und Saugfähigkeit, z. B. Teebeutelpapier der Fa. Schöller und Hösch, 12 g/$cm^2$ Flächengewicht, 50 µm Dicke, 50 ml/m Saugvolumen, imprägniert und 5 Min. bei 30 °C getrocknet. Das Papier wird anschliessend auf 1 cm Breite geschnitten.

B) Oxidationspapier

| | |
|---|---|
| $K_3[Fe(CN)_6]$ | 300 mmol/l |
| in Wasser | |

Mit dieser Lösung wird ein gleichartiges Papier wie das Substratpapier imprägniert, analog getrocknet und auf 6 mm Breite geschnitten.

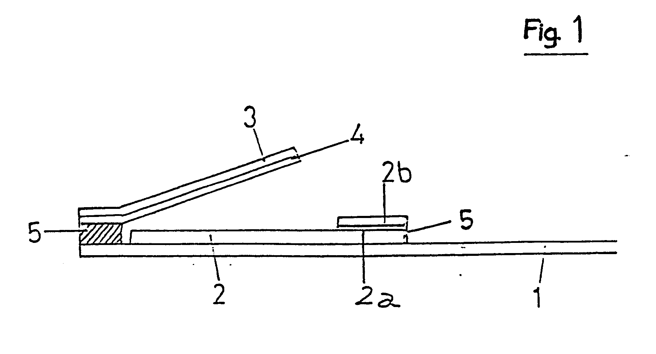

Das Material wird in besonders vorteilhafter Weise in Teststreifen verarbeitet, wie sie in der DOS 3 130 749 beschrieben sind (siehe Fig. 1). Dabei wird eine 1 cm breite, durchsichtige Polycarbonatfolie, 110 µm dick, gemeinsam mit dem Substratpapier einseitig auf einem Plastikstreifen befestigt, so dass die Folie nach aussen kommt. Daneben wird ein 15 mm breites Glasfaservlies, Dicke 1,5 mm, Faserstärke ca. 2 µm, aufgebracht, so dass die freien Enden der Folie und des Substratpapieres noch 6 mm über das Vlies reichen. Bündig mit dem anderen Ende des Glasfaservlieses wird das Oxidationspapier und ein 6 mm breiter Streifen des gleichen Glasfasermaterials auf dem Glasfaservlies befestigt. Dann wird in 6 mm breite Teststreifen geschnitten.

1 Trägerfolie
2 Glasfaservlies, 15 mm breit
2a Oxidationspapier
2b Glasfaservlies, 6 mm breit
3 durchsichtige Folie
4 Substratpapier
5 Kleber

Bringt man nun 30 µl Vollblut auf die Glasfaser 2b, so durchdringt innerhalb 30–60 sec der Plasmaanteil das gesamte Glasfaservlies 2 und hat gleichzeitig das $K_3[Fe(CN)_6]$ aus dem Oxidationspapier gelöst, während die Erythrozyten in und unter 2b festgehalten werden. Durch Andrücken der durchsichtigen Folie kommt nun die γ-GT im Plasma und das Oxidationsmittel mit dem Substratpapier in Berührung, welches gleichmässig durchfeuchtet wird. Es bildet sich je nach Konzentration der γ-GT und der Reaktionszeit eine mehr oder weniger ausgeprägte Grünfärbung, deren Intensitätsänderung proportional zur Enzymaktivität ist und gegebenenfalls in einem Remissionsphotometer zwischen 565–850 nm gemessen werden kann. Natürlich kann auch anderes Probenmaterial wie Plasma, Serum oder sonstiges biologisches Material in gleicher Menge eingesetzt werden.

Diese Anordnung erweist sich besonders deswegen als vorteilhaft, weil in der Phase der Plasmagewinnung Bilirubin bereits zu Biliverdin oxidiert wird. Diese Reaktion würde sonst zusammen mit der γ-GT-Reaktion laufen und, da Biliverdin bei 630 nm absorbiert, die Kinetik stören.

**Patentansprüche**

1. Verfahren zur kinetischen Bestimmung der γ-Glutamyltransferase-Aktivität durch Umsetzung eines als Enzymsubstrat wirkenden γ-Glutamylarylamids mit einem γ-Glutamyl-Akzeptor in Gegenwart von γ-Glutamyltransferase und photometrische Verfolgung der Bildung eines als Folge der enzymatischen Reaktion gebildeten Farbstoffs als Mass für die Aktivität der γ-Glutamyltransferase, dadurch gekennzeichnet, dass das γ-Glutamylarylamid gleichzeitig mit dem zu bestimmenden Enzym, einem γ-Glutamyl-Akzeptor, Ferricyanid oder Peroxid/Peroxidase als Oxidationsmittel und einem als Kupplungskomponente wirkenden aromatischen Amin oder Phenol bei pH 6 bis 10 umgesetzt und hierbei durch enzymatische Reaktion aus dem Enzymsubstrat ein Arylamin freigesetzt wird, das mit der anwesenden Kupplungskomponente oxidativ zu einem Farbstoff gekuppelt wird, welcher ein Absorptionsmaximum oberhalb 565 nm aufweist.

2. Verfahren nach Anspruch 1, dadurch gekennzeichnet, dass als γ-GT-Substrat eine Verbindung der Formel I verwendet wird

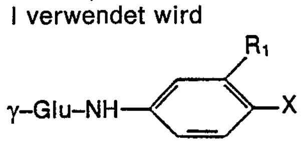 (I)

in der

X die Gruppe $NR_2R_3$ oder $-O-R_2$

$R_1$ Wasserstoff, eine Alkoxy-, Alkyl- oder Arylgruppe mit bis zu 8 C-Atomen, Chlor, Brom, Jod oder die Gruppe $NR_2R_3$, $-SO_3R_2$, $-COOR_2$ und

$R_2$ und $R_3$ gleich oder verschieden sind und Wasserstoff, eine Alkyl- oder Arylgruppe mit bis zu 8 C-Atomen, die auch durch OH, Alkoxy, $-CO_2H$, $SO_3H$ substituiert sein kann darstellen.

3. Verfahren nach Anspruch 1 oder 2, dadurch gekennzeichnet, dass man als Kupplungskomponente eine Verbindung der Formel II verwendet

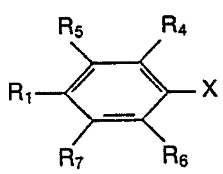 (II)

in der

X eine $NR_2R_3$ oder $-O-R_3$-Gruppe,

$R_1$ Wasserstoff oder Chlor, wenn $R_4$ und/oder $R_6$ auch Chlor sind,

$R_2$ und $R_3$ gleich oder verschieden sind und Wasserstoff, eine Alkyl-, Aralkyl- oder Arylgruppe mit bis zu 8 C-Atomen, die gegebenenfalls auch durch Hydroxy, Alkoxy mit bis zu 5 C-Atomen, $-CO_2H$, $SO_3H$ substituiert sein kann,

$R_4$ und $R_6$ Wasserstoff, niedrig Alkyl mit bis zu 5 C-Atomen, Chlor, Brom, Jod, $-COOR_2$, $SO_3R_2$, $OR_2$, $NR_2R_3$, wobei $R_2$ und $R_3$ die obige Bedeutung haben,

$R_5$ und $R_7$ Wasserstoff oder eine Alkylgruppe mit bis zu 5 C-Atomen bedeuten, wobei

$R_4$ und $R_5$ beziehungsweise $R_6$ und $R_7$ zusammen mit dem Benzolring auch ein Naphthyl- oder Anthrylgerüst bilden können.

4. Verfahren nach einem der Ansprüche 1 bis 3, dadurch gekennzeichnet, dass als Kupplungskomponente 2,3-Xylenol, Diethylmetanilsäure, N-Ethyl-N-(3′-sulfobenzol)-anilin oder N-Methylanthranilsäure verwendet wird.

5. Reagenz zur kinetischen Bestimmung der γ-Glutamyltransferase-Aktivität, dadurch gekennzeichnet, dass es folgende Reagenzien enthält:

einen Puffer mit pH 6–10 in der Konzentration von 10–1000 mmol/l,

ein γ-Glutamylarylamid als Enzymsubstrat in der Konzentration von 1–100 mmol/l,

einen γ-Glutamyl-Akzeptor in der Konzentration von 10–500 mmol/l,

Ferricyanid oder Peroxid/Peroxidase als Oxidationsmittel in der Konzentration von 0,1–500 mmol/l und eine Kupplungskomponente in der Konzentration von 1–500 mmol/l, die mit einem aus dem Enzymsubstrat freigesetzten Arylamin oxidativ zu einem Farbstoff gekuppelt wird, dessen Absorptionsmaximum oberhalb 565 nm liegt.

6. Reagenz gemäss Anspruch 5, dadurch gekennzeichnet, dass die Reagenzien auf einen saugfähigen Träger imprägniert sind.

7. Reagenz gemäss Anspruch 5 oder 6, dadurch gekennzeichnet, dass der Puffer einen pH-Wert von 7,5–8,5 hat.

**Claims**

1. Process for the kinetic determination of γ-glutamyl transferase activity by reaction of a γ-glutamylarylamide acting as enzyme substrate with a γ-glutamyl acceptor in the presence of γ-glutamyl transferase and photometric monitoring of the formation of a colour material formed as a result of the enzymatic reaction as measure for the activity of the γ-glutamyl transferase, characterised in that the γ-glutamylarylamide is reacted simultaneously with the enzyme to be determined, a γ-glutamyl acceptor, ferricyanide or peroxide/peroxidase as oxidation agent and an aromatic amine or phenol acting as coupling component at pH 6 to 10 and an arylamine is hereby liberated by enzymatic reaction from the enzyme substrate. which is coupled oxidatively with the coupling component present to give a coloured material which displays an absorption maximum above 565 nm.

2. Process according to claim 1. characterised in that as γ-GT substrate there is used a compound of the formula I

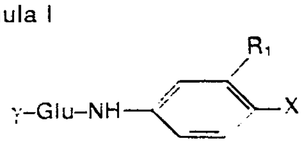 (I)

in which X represents the group $NR_2R_3$ or $-O-R_2$, $R_1$ hydrogen, an alkoxy, alkyl or aryl group with up to 8 C-atoms, chlorine, bromine, iodine or the group $NR_2R_3$, $-SO_3R_2$, $-COOR_2$ and $R_2$ and $R_3$ are the same or different and represent hydrogen, an alkyl or aryl group with up to 8 C-atoms which can also be substituted by OH, alkoxy, $-CO_2H$, $SO_3H$.

3. Process according to claim 1 or 2, characterised in that, as coupling component, one uses a compound of the formula II

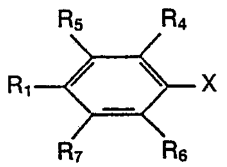 (II)

in which X signifies an $NR_2R_3$ or $-O-R_3$ group, $R_1$ hydrogen or chlorine when $R_4$ and/or $R_6$ are also chlorine, $R_2$ and $R_3$ are the same or different and signify hydrogen, an alkyl, aralkyl or aryl group with up to 8 C-atoms, which can possibly also be substituted by hydroxyl, alkoxy with up to 5 C-Atoms, $-CO_2H$, $SO_3H$, $R_4$ and $R_6$ hydrogen, lower alkyl with up to 5 C-atoms, chlorine, bromine, iodine, $-COOR_2$, $SO_3R_2$, $OR_2$, $NR_2R_3$, whereby $R_2$ and $R_3$ have the above meaning, $R_5$ and $R_7$ signify hydrogen or an alkyl group with up to 5 C-atoms, whereby $R_4$ and $R_5$ or $R_6$ and $R_7$, together with the benzene ring, can also form a naphthyl or anthryl structure.

4. Process according to one of claims 1 to 3, characterised in that, as coupling component, there is used 2,3-xylenol, diethylmetanilic acid, N-ethyl-N-(3′-sulphobenzene)-aniline or N-methylanthranilic acid.

5. Reagent for the kinetic determination of the γ-glutamyl transferase activity, characterised in that it contains the following reagents:

a buffer with pH 6–10 in the concentration of 10–1000 mmole/l.,

a γ-glutamylarylamide as enzyme substrate in the concentration of 1–100 mmole/l.,

a γ-glutamyl acceptor in the concentration of 10–500 mmole/l.,

ferricyanide or peroxide/peroxidase as oxidation agent in the concentration of 0.1–500 mmole/l. and a coupling component in the concentration of 1–500 mmole/l. which is coupled oxidatively with an arylamine liberated from the enzyme substrate to give a coloured material, the absorption maximum of which lies above 565 nm.

6. Reagent according to claim 5, characterised in that the reagents are impregnated on to an absorbent carrier.

7. Reagent according to claim 5 or 6, characterised in that the buffer has a pH value of 7.5–8.5.

**Revendications**

1. Procédé pour la détermination cinétique de l'activité γ-glutamyl-transferase, par réaction d'un γ-glutamyl-arylamide, fonctionnant comme un substrat d'enzyme, avec un γ-glutamyl-accepteur, en présence de γ-glutamyl-transferase, et contrôle photométrique de la formation d'un colorant formé par suite de la réaction enzymatique, comme mesure de l'activité γ-glutamyl-transferase, caractérisé en ce que le γ-glutamyl-arylamide réagit simultanément avec l'enzyme à déterminer, un γ-glutamyl-accepteur, du ferricyanure ou du peroxyde/peroxydase comme agent d'oxydation et une amine aromatique ou un phénol agissant comme composé de copulation, à un pH de 6 à 10, et qu'ainsi, par réaction enzymatique, le substrat d'enzyme libère une arylamine qui, avec le composé de copulation présent, est transformée par copulation oxydative en un colorant qui présente un maximum d'absorption au-dessus de 565 nm.

2. Procédé selon la revendication 1, caractérisé en ce que comme substrat de γ-GT, on utilise un composé de formule I

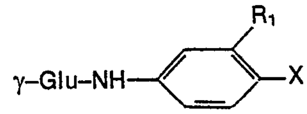 (I)

dans laquelle

X représente le groupe $NR_2R_3$ ou $-O-R_2$

$R_1$ représente un atome d'hydrogène, un groupe alcoxy, alkyle ou aryle avec jusqu'à 8 atomes de carbone, un atome de chlore, de brome ou d'iode, ou les groupes $NR_2R_3-SO_3R_2-COOR_2$ et

$R_2$ et $R_3$ sont identiques ou différents et représentent un atome d'hydrogène, un groupe alkyle ou aryle avec jusqu'à 8 atomes de carbone, qui peuvent être également substitués par un OH, un alcoxy $-CO_2H$, $SO_3H$.

3. Procédé selon la revendication 1 ou 2, caractérisé en ce que comme composé de copulation, on utilise un composé de formule II

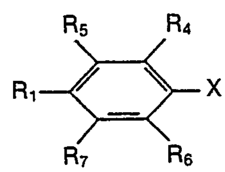 (II)

dans laquelle

X représente un groupe $NR_2R_3$ ou $-O-R_3$,

$R_1$ représente un atome d'hydrogène ou de chlore, lorsque $R_4$ et/ou $R_6$ représentent également un atome de chlore,

$R_2$ et $R_3$ sont identiques ou différents et représentent un atome d'hydrogène, un groupe alkyle, aralkyle ou aryle avec jusqu'à 8 atomes de carbone, qui peuvent être également substitués par un OH, un alcoxy avec jusqu'à 5 atomes de carbone, $CO_2H$, $SO_3H$,

$R_4$ et $R_6$ représentent un atome d'hydrogène, un groupe alkyle inférieur avec jusqu'à 5 atomes de carbone, un atome de chlore, de brome, d'iode, un groupe $COOR_2$, $SO_3R_2$, $OR_2$, $NR_2R_3$, $R_2$ et $R_3$ ayant la même définition que ci-dessus,

$R_5$ et $R_7$ représentent un atome d'hydrogène ou un groupe alkyle avec jusqu'à 5 atomes de carbone,

$R_4$ et $R_5$, ainsi que $R_6$ et $R_7$ pouvant former, en commun avec le cycle benzènique, également un squelette de naphtyle ou d'anthryle.

4. Procédé selon l'une des revendications 1 à 3, caractérisé en ce que comme composé de copulation, on utilise le 2,3-xylènol, l'acide diéthylmétanilique, la N-éthyl-N-(3′-sulfobenzène)-aniline ou l'acide N-méthylanthranilique.

5. Réactif pour la détermination cinétique de l'activité γ-glutamyl-transferase, caractérisé en ce qu'il contient les réactifs suivants:

un tampon avec un pH de 6–10 et à une concentration de 10–1000 mmole/l,

un γ-glutamyl-arylamide comme substrat d'enzyme, à une concentration de 1–100 mmole/l,

un γ-glutamyl-accepteur, à une concentration de 10–500 mmole/l,

du ferricyanure ou du peroxyde/peroxydase comme agent d'oxydation, à une concentration de 0,1–500 mmole/l, et un composé de copulation à une concentration de 1–500 mmole/l, qui est transformé, par copulation oxydative avec une arylamine libérée par le substrat d'enzyme, en un colorant dont le maximum d'absorption est situé au-dessus de 565 nm.

6. Réactif selon la revendication 5, caractérisé en ce que les réactifs sont imprégnés sur un support absorbant.

7. Réactif selon la revendication 5 ou 6, caractérisé en ce que le tampon présente un pH de 7,5–8,5.

Fig. 1